# EUROPEAN PATENT APPLICATION

(11) **EP 0 745 393 A1**
(43) Date of publication of application: **04.12.1996**
(21) Application number: 96108640.2
(22) Date of filing: 30.05.1996
(51) Int. Cl.: A61L 15/28, A61L 15/58, A61F 13/02, B32B 27/06

(54) **Bio-compatible adhesive pad**

(30) Priority: 02.06.1995 JP 159937/95
(71) Applicant: LINTEC Corporation, Tokyo (JP)
(72) Inventor: Okabe, Hideaki, Kanagawa-ken (JP); Sakurai, Satoshi, Chiba-shi, Chiba-ken (JP)
(74) Representative: Türk, Gille, Hrabal, Leifert

(57) **Abstract**

A bio-compatible adhesive pad comprises a laminate containing a layer of a flexible resin and a layer of an adhesive material, and a pad layer compatible with biological surfaces which is laminated on the layer of an adhesive material of the laminate. The bio-compatible adhesive pad absorbs blood and exudates from wound, does not cause ablation of newly formed tissues during exchange of the pads, prevents invasion of bacteria from the outside, can be used easily even by unskilled persons, and is used for protection of wound with excellent safety.

## Description

### FIELD OF THE INVENTION

The present invention relates to a bio-compatible adhesive pad. More particularly, the present invention relates to a bio-compatible adhesive pad which comprises a combination of a pad layer compatible with biological surfaces which absorbs blood and exudates to form a gel by just attaching to the part of wound and an adhesive layer of a flexible resin having an excellent water vapor permeability, prevents secondary infection, has the effect of promoting cure of the wound, and is excellent in easy handling.

### PRIOR ART OF THE INVENTION

Wound is an open injury which is formed by rupture of skin or a mucous membrane by a stimulus from the outside. For treatment to cure the wound, the surface of the wound is sterilized, and when the surface of the wound is large, the surface is sutured together so that the edges of the wound are brought into contact to each other to cure the wound. This treatment has a problem that blood and exudates are attached to a gauze which is applied to the wound, and this causes ablatio of newly formed tissues attached to the solidified blood and exudates when the used gauze is exchanged to a new gauze, resulting in delayed cure. A film dressing is sometimes used alone in place of a gauze. However, this method has a problem that blood and exudates leak from the film dressing when the blood and the exudates are large in the amounts.

### SUMMARY OF THE INVENTION

The present invention accordingly has an object to provide a bio-compatible adhesive pad which absorbs blood and exudates from wound, does not cause ablation of newly formed tissues during exchange of the pads, prevents invasion of bacteria from the outside, can be used easily even by unskilled persons, and is used for protection of wound with excellent safety.

As the result of extensive investigations undertaken by the present inventors to achieve the object described above, it was discovered that a pad comprising a layer of an adhesive material formed on a layer of a flexible resin and a pad layer compatible with biological surfaces which is attached to the layer of an adhesive material absorbs blood and exudates, prevents secondary infection of wound, and promote cure of the wound. The present invention has been completed on the basis of the discovery.

Thus, the present invention comprises:
(1) A bio-compatible adhesive pad comprising a laminate containing a layer of a flexible resin and a layer of an adhesive material, and a pad layer compatible with biological surfaces which is attached to the layer of an adhesive material in the laminate;
(2) A bio-compatible adhesive pad described in (1), wherein the bio-compatible adhesive pad additionally comprises a supporting sheet laminated on the layer of a flexible resin in the laminate and a release sheet laminated on the pad layer compatible with biological surfaces;
(3) A bio-compatible adhesive pad described in any of (1) and (2), wherein the layer of a flexible resin has a 30 % modulus of 50 kg/cm² or less;
(4) A bio-compatible adhesive pad described in any of (1), (2) and (3), wherein the flexible resin is selected from polyurethanes and polyesters;
(5) A bio-compatible adhesive pad described in any of (1), )2), (3) and (4), wherein the laminate containing a layer of a flexible resin and a layer of an adhesive material has a water vapor permeability of 500 g/m^{2.}24 hr or more;
(6) A bio-compatible adhesive pad described in any of (1), (2), (3), (4) and (5), wherein the pad layer compatible with biological surfaces is obtained by forming fiber of alginic acid salts into a sheet;
(7) A bio-compatible adhesive pad described in (6), wherein the fiber of alginic acid salts is reinforced with other fiber materials;
(8) A bio-compatible adhesive pad described in any of (6) and (7), wherein the fiber of alginic acid salts contain a divalent metal, the fraction of the alginic acid salt of the divalent metal in the alginic acid salts is 60 to 95 %, and the divalent metal is one or more types of metal selected from calcium, barium, nickel, and cobalt:
(9) A bio-compatible adhesive pad described in any of (6), (7) and (8), wherein the fiber of alginic acid salts contains an inorganic antimicrobial agent of a metal ion type having an antimicrobial activity; and
(10) A bio-compatible adhesive pad described in (9), wherein the inorganic antimicrobial agent of a metal ion type is obtained by mixing silver and/or zinc with a zeolite-type compound.

### BRIEF DESCRIPTION OF THE DRAWINGS

Figure 1 shows a sectional view of a bio-compatible adhesive pad as an embodiment of the present invention.

Figure 2 shows a sectional view of a bio-compatible adhesive pad as another embodiment of the present invention.

Figure 3 shows a sectional view of a bio-compatible adhesive pad as still another embodiment of the present invention.

The numbers in the figures have the meanings as listed in the following:
- 1: a layer of a flexible resin
- 2: a layer of an adhesive material
- 3: a pad layer compatible with biological surfaces
- 4: a release sheet
- 5: a supporting sheet

### DETAILED DESCRIPTION OF THE INVENTION

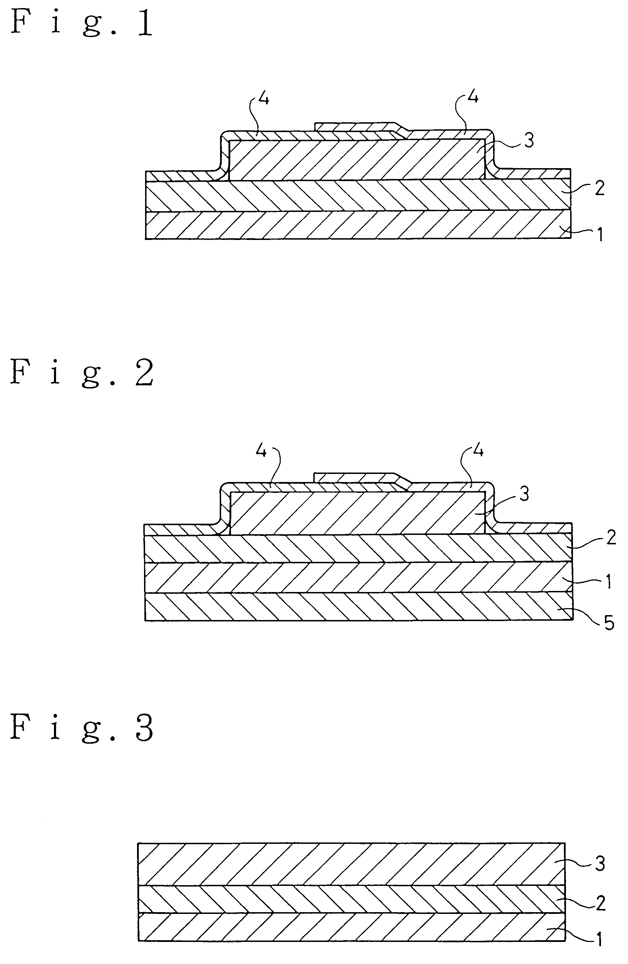

Figure 1 shows a sectional view of a bio-compatible adhesive pad as an embodiment of the present invention. This bio-compatible adhesive pad comprises a laminate containing a layer of a flexible resin 1 and a layer of an adhesive material 2, a pad layer compatible with biological surfaces 3 attached to the surface of the layer of an adhesive material in such a manner that a part of the surface of the layer of an adhesive material is left exposed, and a release sheet 4 which covers the exposed part of the surface of the layer of an adhesive material and the pad layer compatible with biological surfaces. When the bio-compatible adhesive pad is applied to wound, the release sheet is removed, and the laminate comprising the flexible resin, the layer of an adhesive, and the pad layer compatible with biological surfaces is attached to the skin by the layer of an adhesive material in such as manner that the pad layer compatible with biological surfaces is brought into contact with the wound.

Figure 2 shows a sectional view of a bio-compatible adhesive pad as another embodiment of the present invention. This bio-compatible adhesive pad comprises a supporting sheet 5, a layer of a flexible resin 1, a layer of an adhesive material 2 laminated on the supporting sheet, a pad layer compatible with biological surfaces 3 attached to the surface of the layer of an adhesive material in such a manner that a part of the surface of the layer of an adhesive material is left exposed, and a release sheet 4 which covers the exposed part of the surface of the layer of an adhesive material and the pad layer compatible with biological surfaces. When the bio-compatible adhesive pad is applied to wound, the release sheet is removed, and the laminate comprising the layer of a flexible resin, the layer of an adhesive material, and the pad layer compatible with biological surfaces is attached to the skin by the layer of an adhesive material in such a manner that the pad layer compatible with biological surfaces is brought into contact with the wound. Subsequently, the supporting sheet 5 is removed.

Figure 3 shows a sectional view of a bio-compatible adhesive pad as still another embodiment of the present invention. This bio-compatible adhesive pad comprises a laminate containing a layer of a flexible resin 1 and a layer of an adhesive material 2, and a pad layer compatible with biological surfaces attached to the whole surface of the layer of an adhesive material. When the bio-compatible adhesive pad is applied to wound, the laminate containing the layer of a flexible resin, the layer of an adhesive material, and the pad layer compatible with biological surfaces is attached to skin in such a manner that the pad layer compatible with biological surfaces is brought into contact with the wound, and the laminate is fixed to the skin by a pressure-sensitive adhesive tape, a pressure-sensitive adhesive sheet, or a bandage.

When a supporting sheet is used in the bio-compatible adhesive pad of present invention, the supporting sheet can work as the base sheet for forming the layer of a flexible resin in the process for producing the bio-compatible adhesive pad. The bio-compatible adhesive pad can also be kept in its shape and handled more easily until the adhesive pad is used. The material of the supporting sheet is not particularly limited. For example, a polyester film or a polypropylene film having a thickness of 20 to 80 µm can be used. The method of forming the layer of a flexible resin on the supporting sheet is not particularly limited. For example, a sheet of a flexible resin which has been prepared separately can be laminated to the supporting sheet. As another example, the supporting sheet is coated with a solution of a flexible resin, and the solvent is removed by vaporization to form the layer of a flexible resin. As still another example, the layer of a flexible resin is formed by melt extrusion of a flexible resin on the supporting sheet.

In the present invention, the layer of a flexible resin holds the pad layer compatible with biological surfaces through the layer of an adhesive material and protects the wound from invasion of bacteria from the outside to prevent delayed cure caused by the infection. In the present invention, the layer of a flexible resin preferably has a 30 % modulus of 50 kg/cm² or less, more preferably 30 kg/cm² or less. When the layer of a flexible resin has a 30 % modulus of 50 kg/cm² or less, the bio-compatible adhesive pad is provided with a sufficient flexibility so that the skin is not physically irritated or edema is not formed by friction with the skin, and the bio-compatible adhesive pad can be deformed in conformity with deformation of the skin at any location of the attachment on the skin. The material of the flexible resin is not particularly limited, and a polyurethane resin, a flexible polyester resin, or a polyether polyamide resin can be used. A cellular polyolefin resin may also be used within the range that the barrier property against bacteria and permeability of water vapor and oxygen are not adversely affected. Among these resins, a polyurethane resin or a flexible polyester resin is preferably used. The 30 % modulus is obtained as follows: a film of the resin used for the layer of a flexible resin is prepared; a stress-strain curve of the prepared film is obtained by using a universal tensile tester under the condition of a speed of elongation of 200 mm/min, a width of the test piece of 15 mm, and a distance between benchmarks for elongation of 100 mm, under an atmosphere of 20°C and 65 % RH; the stress at the elongation of 30 % was obtained; and the obtained value is converted to a value expressed by the unit of kg/cm². The thickness of the layer of a flexible resin is not particularly limited. The layer of a flexible resin having a thickness of 10 to 80 µm is preferably used.

In the present invention, the layer of an adhesive material holds the pad layer compatible with biological surfaces to the layer of a flexible resin by adhesion. In the bio-compatible adhesive pads shown in Figures 1 and 2, the layer of an adhesive material also holds the bio-compatible adhesive pads to the skin by the adhesive force. The adhesive material is not particularly limited, and a conventional adhesive material showing quite low skin irritation and heretofore used for adhesive plasters can be used. Examples of the adhesive material include acrylic adhesive materials, rubber adhesive materials, silicone adhesive materials, vinyl ether adhesive materials, and urethane adhesive materials. In the bio-compatible adhesive pad of the present invention, the thickness of the layer of an adhesive material is not particularly limited as long as the water vapor permeability is not adversely affected and the pad layer compatible with biological surfaces can be held attached to the layer of a flexible resin. In the case of the bio-comptaible adhesive pads shown in Figures 1 and 2, it is also necessary that the pad layer compatible with biological surfaces be held attached to the human skin by the layer of an adhesive material. The thickness of the layer of an adhesive material is generally 10 to 50 µm. In the bio-comptaible adhesive pad of the present invention, the water vapor permeability of the laminate of the layer of a flexible resin and the layer of an adhesive material is preferably 500 g/m^{2.}24 hr or more, more preferably 800 g/m^{2.}24 hr. When the water vapor permeability of the laminate of the layer of a flexible resin and the layer of an adhesive material is 500 g/m^{2.}24 hr or more, an eruption of the skin is not likely to occur, and water in blood and exudates from the wound can diffuse through the layer of an adhesive material and the layer of a flexible resin to be vaporized into the air.

In the present invention, the pad layer compatible with biological surfaces is preferably a pad layer prepared by forming fiber of alginic acid salts into a layer. The fiber of alginic acid salts can be obtained by extruding an aqueous solution of an alginic acid salt such as sodium alginate, which can be obtained by extraction of see weeds such as tangle and undaria pinnatifida (wakame), into an aqueous solution of a salt of a multivalent metal such as calcium chloride, to spin the alginic acid salt into fiber by forming an insoluble salt of alginic acid, and subsequently dewatering the obtained fiber.

In the present invention, it is preferred that the fiber of alginic acid salts contains an alginic acid salt of a divalent metal and the fraction of the alginic acid salt of the divalent metal in the alginic acid salts is 60 to 95 %, more preferably 70 to 90 %. The fraction of an alginic acid salt of a divalent metal in the alginic acid salts means the fraction of the carboxyl groups of alginic acid forming the salt of the divalent metal in the total carboxyl groups of alginic acid. More specifically, the fraction can be expressed as the ratio in percentage of the amount of the metal which actually substitutes the hydrogen atom in the carboxyl group of alginic acid to the theoretical amount of the metal when the hydrogen atom in the carboxyl group of alginic acid is substituted with the metal up to 100 %. When the fraction of an alginic acid salt of a divalent metal in the alginic acid salts is more than 95 %, the fiber of alginic acid salts tends to be fragile, and there is the possibility that the fiber is easily broken. When the fraction of an alginic acid salt of a divalent metal in the alginic acid salts is less than 60 %, elements in the fiber of alginic acid salts tends to stick to each other, and there is the possibility that the fiber has inferior flexibility. The process for forming the fiber of alginic acid salts into a sheet is not particularly limited, and conventional wet or dry processes for producing non-woven fabrics can be used. In the process for producing non-woven fabrics, other fiber materials may additionally be used for reinforcement by forming a composite material. Examples of the other fiber material include non-woven fabrics of rayon, polyester, and polypropylene. By forming a composite material as described above, properties suitable for processing, such as the shape holding property during a process, can be provided.

In the present invention, any divalent metal which forms an insoluble salt of alginic acid can be used without particular restriction as the divalent metal used for forming the fiber of alginic acid salts. Among such divalent metals, calcium, barium, nickel, and cobalt are particularly preferably used. The divalent metal can be used singly or as a combination of two or more types.

In the present invention, the fiber of alginic acid salts may contain an inorganic antimicrobial agent of a metal ion type having an antimicrobial activity. The inorganic antimicrobial agent of a metal ion type is not particularly limited as long as the antimicrobial agent has the bactericidal or antimicrobial activity. Inorganic antimicrobial agents of a metal ion type which contain silver, copper, or zinc as the metal ion having the antimicrobial activity and a support, such as zeolite, calcium phosphate, zirconium phosphate, amorphous silicon oxide, and dissolving glass can preferably be used. Among these inorganic antimicrobial agents of a metal ion type, inorganic antimicrobial agents of a metal ion type prepared by mixing silver and/or zinc with zeolite are more preferably used. The method of incorporating the inorganic antimicrobial agents of a metal ion type into the fiber of alginic acid salts is not particularly be limited. It is preferred that a metal ion having the antimicrobial activity is mixed with zeolite, and the obtained mixture of the metal ion having the antimicrobial activity and zeolite is incorporated into the fiber of alginic acid salts. Any of natural zeolite and synthetic zeolite can be used as the zeolite. The particle diameter of the zeolite is not particularly limited, and is preferably 1 to 50 µm. When the particle diameter of the zeolite is less than 1 µm, there is the possibility that workability in handling is inferior. When the particle diameter of the zeolite is more than 50 µm, there is the possibility that the spinning property in the preparation of the fiber is inferior. It is preferred that the metal ion having the antimicrobial activity is mixed with zeolite in such an amount that the content of silver ion in zeolite particles is 2.0 to 3.0 % by weight and the content of zinc ion in zeolite particles is 13 to 16 % by weight.

In the present invention, the fiber of alginic acid salts having the antimicrobial activity can be obtained by adding a zeolite-type compound which contains a metal ion having the antimicrobial activity into an aqueous solution of an alginic acid salt, such as sodium alginate, and subsequently spinning the obtained mixture to fiber by extrusion into an aqueous solution of a divalent metal salt. It is preferred that the zeolite-type compound which contains a metal ion having the antimicrobial activity is added in such an amount that the content of the zeolite-type compound containing the metal ion in the fiber of alginic acid salts is 0.01 to 15.0 % by weight. When the zeolite-type compound which contains a metal ion having the antimicrobial activity is added in such an amount that the content of the zeolite-type compound containing the metal ion in the fiber of alginic acid salts is less than 0.01 % by weight, there is the possibility that the antimicrobial activity of the obtained fiber is insufficient. When the zeolite-type compound which contains a metal ion having the antimicrobial activity is added in such an amount that the content of the zeolite-type compound containing the metal ion in the fiber of alginic acid salts is more than 15.0 % by weight, there is the possibility that preparation of the fiber by spinning becomes difficult. As the fiber having the antimicrobial activity, fiber described in Japanese Patent Application Laid-Open No. Heisei 4(1992)-146218 is preferable.

In the bio-compatible adhesive pads of the present invention shown in Figures 1 and 2, exposed parts of the pad layer compatible with biological surfaces and the layer of an adhesive material are covered with a release sheet. The material of the release sheet is not particularly limited, and a release sheet obtained by coating the surface of a polyester film, a polypropylene film, or paper with a releasing agent, such as a silicone, can be used. The release sheet protects the exposed parts of the pad layer compatible with biological surfaces and the layer of an adhesive material until the bio-compatible adhesive pad is used, and is peeled off before the bio-compatible adhesive pad is used.

When the bio-compatible adhesive pad of the present invention is applied to the surface of wound, the bio-compatible adhesive pad exhibits the excellent function that the surface of wound is protected by absorption of blood and exudates and consequent formation of gel in the pad and the formation of the new skin is promoted, and shows the excellent effect that the necessity of removing the formed gel at the time of exchange of the pads is eliminated by the excellent bio-compatibility. The bio-compatible adhesive pad also protects the wound from invasion of bacteria from the outside by the layer of a flexible resin and prevents delayed cure caused by the secondary infection. Suppressing propagation of bacteria in blood or exudates in the protecting material is enabled by addition of an antimicrobial agent, and cure of wound is promoted. "Curing wound by drying" has heretofore been the practice. However, an environment which is moist to a suitable extent is necessary for a new cell. When the bio-compatible adhesive pad of the present invention is used, while an advantageous moist environment is maintained by the fiber of alginic acid salts by absorbing blood and exudates and forming a gel, the excess amount of water, such as water formed by sweating, is removed to the outside through the layer of a flexible resin, and the formation of new skin is promoted on the surface of wound.

To summarize the advantages of the present invention, the bio-compatible adhesive sheet of the present invention can easily be handled, and provides an excellent effect of promoting cure because the adhesive pad is prepared by laminating a bio-compatible pad layer which can absorb blood and exudates just by attaching to the wound, a layer of an adhesive material which shows quite low skin irritation, and a layer of a flexible resin which allows permeation of oxygen and water vapor and protects the wound from bacteria. When an antimicrobial agent is added, propagation of bacteria is suppressed and a still more excellent effect of promoting cure can be obtained.

The present invention is described in more detail in the following with reference to examples.

### Example 1

A 5 % by weight aqueous solution of sodium alginate (a product of Kimitsu Kagaku Kogyo Co., Ltd.) was prepared. The prepared solution was extruded into a 5 % by weight aqueous solution of calcium chloride through nozzle holes of 0.1 mm diameter to convert sodium alginate soluble in water into an alginic acid salt insoluble in water. The formed alginic acid salt insoluble in water was formed into a white fibrous material by dewatering with ethanol. The obtained fibrous material was cut to the length of 52 mm to obtain fiber of alginic acid salts. The fraction of the alginic acid salt of calcium in the obtained fiber of alginic acid salts was 82.5 %.

The fiber of alginic acid salts obtained above was uniformly laid on a non-woven fabric of rayon. By the treatment of needle punching of the formed material, a non-woven fabric of the alginic acid salts which was reinforced with the non-woven fabric of rayon on one side was obtained. The obtained non-woven fabric laminate had basis weights of 30 g/m² in the part of rayon and 120 g/m² in the part of the fiber of alginic acid salts.

A polyester film of a thickness of 20 µm which had been treated with peeling was used as the supporting sheet, and a sheet of a polyurethane resin (a product of Seiko Kasei Co., Ltd.) having a thickness of 30 µm and a 30 % modulus of 21 kg/cm² was laminated on the supporting sheet as the layer of a flexible resin. Separately, a release sheet was uniformly coated with an acrylic adhesive material (Primal N580; a product of Nippon Acryl Kagaku Co., Ltd.) in such an amount that the dried thickness is 20 µm, and the adhesive material was dried to form a layer of the adhesive material. The sheet of the polyurethane resin laminated to the supporting sheet in the above and the layer of the adhesive material formed above were attached to each other, and then the release sheet was removed. A portion of the prepared laminate was cut off as a sample. The polyester film was removed from the sample, and the water vapor permeability of the laminate composed of the sheet of the polyurethane resin as the layer of a flexible resin and the layer of the adhesive material was measured. The water vapor permeability was found to be 1,090 g/m^{2.}24 hr.

On the layer of the adhesive material of the laminate composed of the polyester film as the supporting sheet, the sheet of the polyurethane as the layer of a flexible resin, and the layer of the adhesive material, the above-prepared non-woven fabric laminate composed of the non-woven fabric of the alginic acid salts and the non-woven fabric of rayon was laminated in such a manner that the side of the non-woven fabric of rayon in the non-woven fabric laminate was brought into contact with the layer of the adhesive material except for a part of the layer of the adhesive material which was left exposed. The obtained laminate was covered with a release sheet of paper to obtain a bio-compatible adhesive pad (Figure 2).

The backs of 10 Wistar rats having weights of 180 to 200 g were each shaved, and wound of loss of skin was surgically formed on each back. The supporting sheet and the release sheet of the bio-compatible adhesive pad prepared above were removed, and the bio-compatible adhesive pad was attached to the wound in such a manner that the non-woven fabric of the fiber of alginic acid salts was brought into contact with the wound. The bio-compatible adhesive pad attached to the back of each Wistar rat was removed after 10 days, and the condition of the wound was visually observed. The skin had recovered to a good condition, and thus the advantageous effect on cure could be confirmed.

### Example 2

The same procedures as those in Example 1 were conducted except that a sheet of a flexible polyester resin (a product of Nichigo Film Co., Ltd.; Flecron) having a thickness of 30 µm and a 30 % modulus of 26 kg/cm² was used in place of the sheet of the polyurethane resin used in Example 1. The water vapor permeability of the laminate composed of the sheet of the flexible polyester resin as the layer of a flexible resin and the layer of the adhesive material was 820 g/m^{2.}24 hr. The skin of Wistar rats had recovered to a good condition after 10 days, and thus the advantageous effect on cure could be confirmed.

### Example 3

An aqueous solution containing 5 % by weight of sodium alginate (a product of Kimitsu Kagaku Kogyo Co., Ltd.) and 0.15 % by weight of Zeomic (a product of Shinagawa Nenryo Co., Ltd.) as an inorganic antimicrobial agent of a metal ion type was prepared as the material solution for spinning. The prepared material solution for spinning was extruded into a 5 % by weight solution of calcium chloride through nozzle holes of 0.1 mm diameter. The sodium alginate soluble in water was converted into an alginic acid salt insoluble in water, and a fibrous material was formed. The formed fibrous material was cut to the length of 52 mm to obtain fiber of alginic acid salts containing Zeomic. The fraction of the alginic acid salt of calcium in the obtained fiber was 82.5 %.

A bio-compatible adhesive pad was prepared by using the fiber of alginic acid salts prepared above in accordance with the same procedures as those in Example 1, and the test on cure of wound was conducted by using Wistar rats also in accordance with the same procedures as those in Example 1. The skin of the Wistar rats had recovered to a good condition after 10 days, and thus the advantageous effect on cure could be confirmed.

### Example 4

A laminate composed of a polyester film as the supporting sheet, a sheet of a polyurethane resin as the layer of a flexible resin, and a layer of an adhesive material was prepared in accordance with the same procedures as those in Example 1. On the whole surface of the prepared laminate, a non-woven fabric laminate composed of the non-woven fabric of the alginic acid salts and the non-woven fabric of rayon which was obtained in accordance with the same procedures as those in Example 1 was laminated in such a manner that the non-woven fabric of rayon in the non-woven fabric laminate was brought into contact with the layer of the adhesive material. Then, the supporting sheet was removed from the prepared laminate to obtain a bio-compatible adhesive pad (Figure 3).

In accordance with the same procedures as those in Example 1, the backs of ten Wistar rats having weights of 180 to 200 g were each shaved, and wound of loss of skin was surgically formed on each back. The bio-compatible adhesive pad was attached to the wound in such a manner that the non-woven fabric of the fiber of alginic acid salts was brought into contact with the wound, and then fixed by a bandage. The bio-compatible adhesive pad attached to the back of each Wistar rat was removed after 10 days, and the condition of the wound was visually observed. The skin had recovered to a good condition, and thus the advantageous effect on cure could be confirmed.

### Comparative Example 1

The backs of ten Wistar rats having weights of 180 to 200 g were each shaved, and wound of loss of skin was surgically formed on each back. The wound was covered with a gauze which was in accordance with The Pharmacopoea of Japan, and the gauze was then fixed by a bandage. The said gauze attached to the back of each Wistar rat was removed after 10 days, and the condition of the wound was visually observed. Adhesion between the wound and the gauze took place to a great extent, and wound was formed again when the gauze was removed. Thus, the advantageous effect on cure could not be found.

### Comparative Example 2

The same procedures as those in Comparative Example 1 was conducted except that the non-woven fabric laminate composed of the non-woven fabric of rayon and the non-woven fabric of the fiber of alginic acid salts which was prepared in Example 1 was used in place of the gauze which was in accordance with The Pharmacopoea of Japan, and the wound was covered with the non-woven fabric of the fiber of alginic acid salts in the non-woven fabric laminate. The non-woven fabric of alginic acid salts attached to the back of each Wistar rat was removed after 10 days, and the condition of the wound was visually observed. The advantageous effect on cure was found, but the recovery of the skin was inferior to that in Examples.

## Claims

1. A bio-compatible adhesive pad comprising a laminate containing a layer of a flexible resin and a layer of an adhesive material, and a pad layer compatible with biological surfaces which is attached to the layer of an adhesive material in the laminate.

2. A bio-compatible adhesive pad according to Claim 1, wherein the bio-compatible adhesive pad additionally comprises a supporting sheet laminated on the layer of a flexible resin in the laminate and a release sheet laminated on the pad layer compatible with biological surfaces.

3. A bio-compatible adhesive pad according to any of Claims 1 and 2, wherein the layer of a flexible resin has a 30 % modulus of 50 kg/cm² or less.

4. A bio-compatible adhesive pad according to any of Claims 1, 2 and 3, wherein the flexible resin is selected from polyurethanes and polyesters.

5. A bio-compatible adhesive pad according to any of Claims 1, 2, 3 and 4, wherein the laminate containing a layer of a flexible resin and a layer of an adhesive material has a water vapor permeability of 500 g/m^{2.}24 hr or more.

6. A bio-compatible adhesive pad according to any of Claims 1, 2, 3, 4 and 5, wherein the pad layer compatible with biological surfaces is obtained by forming fiber of alginic acid salts into a sheet.

7. A bio-compatible adhesive pad according to Claim 6, wherein the fiber of alginic acid salts is reinforced with other fiber materials.

8. A bio-compatible adhesive pad according to any of Claims 6 and 7, wherein the fiber of alginic acid salts contain a divalent metal, the fraction of the alginic acid salt of the divalent metal in the alginic acid salts is 60 to 95 %, and the divalent metal is one or more types of metal selected from calcium, barium, nickel, and cobalt.

9. A bio-compatible adhesive pad according to any of Claims 6, 7 and 8, wherein the fiber of alginic acid salts contains an inorganic antimicrobial agent of a metal ion type having an antimicrobial activity.

10. A bio-compatible adhesive pad according to Claim 9, wherein the inorganic antimicrobial agent of a metal ion type is obtained by mixing silver and/or zinc with a zeolite-type compound.
